# EUROPEAN PATENT SPECIFICATION

(11) **EP 0 827 422 B1**
(45) Date of publication and mention of the grant of the patent: **02.01.2002**
(21) Application number: 96915780.9
(22) Date of filing: 14.05.1996
(51) Int. Cl.: B01J 23/52, B01J 23/656, B01J 23/68, C07C 67/055

(54) **CATALYST FOR VINYL ACETATE MANUFACTURE**
KATALYSATOR ZUR HERSTELLUNG VON VINYLAZETAT
CATALYSEUR POUR LA FABRICATION DE L'ACETATE DE VINYLE

(30) Priority: 23.05.1995 US 449604
(43) Date of publication of application: 11.03.1998
(73) Proprietor: HOECHST CELANESE CORPORATION, Somerville, NJ 08876-1258 (US)
(72) Inventor: ABEL, Roland, D-46147 Oberhause (DE); NICOLAU, Ioan, Corpus Christi, TX 78413 (US)
(74) Representative: James, Anthony Christopher W.P.
(86) International application number: US9606848
(87) International publication number: WO9637294

(56) References cited:
- EP-A- 0 417 867
- EP-A- 0 634 209
- EP-A- 0 672 453

## Description

### BACKGROUND OF THE INVENTION

### Field of the Invention

The present invention relates to a novel method which results in a novel catalyst useful in the reaction of ethylene, oxygen, and acetic acid in the vapor phase to form vinyl acetate. The catalyst comprises metallic palladium, gold, and, in addition, at least one metal of the group consisting of rhenium and zirconium deposited on a suitable porous support.

### Description of the Prior Art

It is known in the art that vinyl acetate can be produced by reacting ethylene, oxygen, and acetic acid in the gaseous phase and in the presence of a catalyst comprising palladium, gold, and an alkali metal acetate supported on certain carrier materials such as silica. Such catalyst systems exhibit an acceptable activity and, generally, low selectivity to by-products, such as carbon dioxide, ethyl acetate, and heavy ends.

Generally, patents describing the manufacture of catalysts for the commercial manufacture of vinyl acetate from acetic acid, ethylene, and oxygen describe and claim various methods for the deposition of the metallic components, i.e. palladium, gold, cadmium, and the like, to form a narrow band of metal at the surface of the catalyst support. Several of the more pertinent vinyl acetate catalysts and their methods of manufacture are discussed below.

U.S. Pat. No. 3,190,912 discloses the preparation of catalysts for preparing unsaturated organic esters wherein the catalyst consists of unsupported or supported metals of the platinum or palladium group or oxides or salts thereof, either organic or inorganic. Supported catalyst is prepared by dissolving the metal salt or salts in, for example, water, adding the support and evaporating the solvent. The noble metal is distributed uniformly throughout the support. According to the patent, the activity of the catalyst can be promoted by the addition of a metal halide promoter.

U.S. Pat. No. 3,275,680 discloses a palladium catalyst on aluminum oxide support which when promoted with alkali acetate has been found suitable for the production of organic acetates, especially vinyl acetate. The palladium catalyst is deposited throughout the catalyst support. There is no specific disclosure in the patent of combinations of two noble metals or of the use of alloys. The catalyst is prepared by impregnating the support with an aqueous solution of the noble metal salt and precipitating the noble metal on the support by reduction.

U.S. Pat. No. 3,743,607 discloses a catalyst containing palladium, gold, and an alkali metal acetate. The catalyst is prepared by impregnating a carrier such as silicic acid, aluminum oxide, or silicate, aluminum phosphate, etc. with an aqueous solution of a palladium salt and of a gold salt and evaporating the resulting mixture to dryness. The palladium and gold salts are reduced to their metallic state by a reducing agent. The catalyst is then washed with water, impregnated with a solution of sodium acetate and upon drying is ready for use.

British Pat. No. 1,333,449 describes a palladium-gold catalyst suitable for the preparation of vinyl acetate. It can be established that the process of this patent results in the palladium salts being deposited throughout the catalyst support. The catalyst is prepared by impregnating the catalyst support with a solution of palladium acetate, barium aceto-aurate and potassium acetate in acetic acid and subsequently drying it. The catalyst is disclosed to be a palladium salt and other additives, such as gold, gold salts, alkaline earth metal salts, and alkaline metal salts supported on a carrier.

Published Japanese Patent Application No. 48-10135/1973 describes a process for preparing surface impregnated supported vinyl acetate catalyst. In a preliminary step a small amount of reduced metal (such as gold) is deposited throughout the porous support. This is followed by impregnation of the required amount of palladium catalyst which then is deposited on the surface around the preformed metal particles. The palladium catalyst is deposited as a surface layer which has a thickness of about 15% or less of the particle ratios. The use of alkali metal acetate salts as a co-catalyst is recommended.

British Patent Specification 1,283,737 discloses a method of preparing a supported metal catalyst by contacting the support with a solution of a compound of a metal such as platinum, palladium, etc. and converting the deposited compound to the metallic state, the porous catalyst support having been impregnated with an alkaline solution and saturated with from 25 to 90% of water or an alcohol. The degree of penetration of metal into the catalyst support pellet is disclosed to be up to 50% of the pellet radius. The use of a minor amount of an activator, such as sodium and potassium acetate, is recommended.

U.S. Pat. No. 3,939,199 discloses the use of a catalyst wherein palladium is deposited on a catalyst support which has a pore volume of from 0.4 to 1.0 ml./g. and wherein less than 10% of the total pore volume is attributable to micropores having a diameter of less than 30 Angstrom. The usual inert substances such as silicic acid, silicates, aluminum silicates, titanium oxide, zirconium oxide, and various glasses are disclosed as the suitable support materials. For the preparation of vinyl acetate from ethylene and oxygen, the catalyst support is impregnated with a solution of palladium acetate, cadmium acetate, and potassium acetate in acetic acid with subsequent drying.

U. S. Patent 3,822,308 describes the manufacture of a vinyl acetate catalyst containing 0.6-6.0 grams/liter of palladium and 0.1-3.0 grams/liter of gold on a silica support wherein the adsorbed metal salts were precipitated on the catalyst support by use of an aqueous solution of sodium hydroxide in a quantity equal to 1-110% of the absorptive capacity of the support.

U. S. 4,048,096 describes the manufacture of a vinyl acetate catalyst wherein the dissolved metal salts were absorbed on the support from a solution equal to the pore volume of the support by mechanically tumbling the supports in a rotating vessel. The salts were then fixed with alkali without drying the salt-loaded support.

U. S. 4,087,622 discloses a palladium-gold catalyst manufactured by a two-step impregnation and precipitation process.

U. S. 3,775,342 describes a method of catalyst preparation generally called pore volume impregnation. The patent discloses a vinyl acetate catalyst which provides an interior band of palladium-gold alloy deposited on the catalyst support. The catalyst is disclosed to be prepared by treating the catalyst support simultaneously or successively, with or without intermediate drying, with a solution of palladium and gold salts and a solution which contains compounds that are able to react on the catalyst support with the palladium-gold salts to form water insoluble palladium and gold compounds and then transforming the water insoluble palladium and gold compounds into the noble metals by treatment with reducing agents, and removing the water soluble compounds by washing. It is recommended to employ an alkali metal carboxylate, e.g., alkali metal acetate, on the catalyst such that after drying the catalyst contains from 1 to 30 percent by weight alkali metal carboxylate. In all of the examples, in which the catalyst support was treated successively with the solution of palladium and gold compounds and the precipitating solution the catalyst was dried between each of the successive treatments. In pore volume impregnation the amount of solution containing a salt of the precious metal is approximately equal to the pore volume of the porous support. In that patent there is disclosed a list of other metals which may be coprecipitated with the gold and palladium, including copper. There is, however, no indication in the disclosure of an example of how to make a catalyst containing copper as a third metal species or that the presence of copper is advantageous, either to the make-rate of vinyl acetate or to the selectivity towards ethyl acetate, carbon dioxide or other impurities. It appears that the inventors were merely speculating that any of the sixteen or more metals disclosed in any concentration could exist in the final catalyst.

Other patents and publications of which the inventors are aware describe catalysts for the vapor phase reaction of acetic acid, ethylene, and oxygen to form vinyl acetate and which contain copper as a reduced metal component. These catalysts, however, do not contain any gold component. These include U. S. 3,759,839; U. S. 3,641,121; U. S. 3,671,576; French Patents 1,566,972 and 1,583,899; Japanese Patent Application (Kokoku) 54-8638; Japan 46033013 (Chemical Abstracts 76(8):34719a; Japan 46032644 (Chemical Abstracts 76(8):34718z; and Revista de Chemie Vol. 26, No. 9, pages 719-723 (1975).

Several new patents have issued directed to improved methods and to the resulting product. Examples of these patents are U. S. 5,332,710 and U. S. 5,314,858 directed to new methods of manufacture of catalysts containing palladium and gold.

In accordance with U. S. 5,314,858, a useful catalyst is formed by the method of U. S. Patent 3,775,342 wherein the metals as salts thereof are combined and are adsorbed into the pores of the catalyst by the pore volume impregnation method. (1) simultaneously or successively impregnating a catalyst support with aqueous solutions of palladium and gold salts such as sodium-palladium chloride and auric chloride, (2) fixing the precious metals on the support by precipitating water-insoluble palladium and gold compounds by treatment of the impregnated supports with a reactive basic solution such as aqueous sodium hydroxide which reacts to form hydroxides of palladium and gold on the support surface, (3) washing with water to remove the chloride ion (or other anion), and (4) reducing the precious metal hydroxides to free palladium and gold, wherein the improvement comprises during fixing step (2) utilizing two separate precipitation stages wherein the amounts of the reactive compound in contact with the salt-impregnated support in each stage is no more than that required to react with the water soluble precious metal compounds impregnated in the support. Between the separate fixing or precipitation stages, the support which has been impregnated with the reactive basic solution is allowed to stand for a set period of time to allow precipitation of the water insoluble precious metal compounds before the second fixing stage in which additional reactive basic compound is added to the support.

U. S. 5,332,710 discloses (1) simultaneously or successively impregnating a catalyst support with aqueous solutions of palladium and gold salts such as sodium-palladium chloride and auric chloride, (2) fixing the precious metals on the support by precipitating water-insoluble palladium and gold compounds by immersion of the impregnated supports in a reactive basic solution such as aqueous sodium hydroxide which reacts to form hydroxides of palladium and gold on the support surface, (3) washing with water to remove the chloride ion (or other anion), and (4) reducing the precious metal hydroxides to free palladium and gold, wherein the improvement comprises during fixing step (2) rotating the impregnated catalyst supports while such impregnated supports are immersed in the reaction solution at least during the initial precipitation period and prior to letting the treated catalyst stand for an extended period of time to continue the precipitation of the water-insoluble palladium and gold compounds.

U. S. 5,194,417 describes a process for baking the virgin catalyst in an inert atmosphere for at lease 15 minutes to improve selectivity of the catalyst toward the manufacture of vinyl acetate.

U. S. 5,185,308 claims a palladium-gold catalyst with a defined ratio of precious metals among other defined parameters. According to the disclosure, the catalyst consists essentially of (1) a catalyst support having a particle diameter of from about 3 to about 7 mm and a pore volume of from 0.2 to about 1.5 ml/g, (2) gold and palladium in a ratio in the range 0.60 to 1.25 distributed in the outermost 1.0 mm thick layer of the catalyst support particles, and (3) from about 3.5 to about 9.5% by weight of potassium acetate.

As can be appreciated by the above examples of the prior art, the basic method of forming the vinyl acetate catalyst containing palladium and gold deposited on a catalyst support has evolved to one which comprises (1) impregnating the support with aqueous solutions of water-soluble palladium and gold compounds, (2) precipitating water-insoluble palladium and gold compounds on the catalyst support by contacting the impregnated catalyst support with a solution of compounds capable of reacting with the water-soluble palladium and gold compounds to form the insoluble metal compounds (3) washing the treated catalyst with water to remove anions which are freed from the initially impregnated palladium and gold compounds during precipitation and (4) converting the water-insoluble palladium and gold compounds to the free metal by treatment with a reducing agent. A final treatment usually involves (5) impregnating the reduced catalyst with an aqueous alkali metal acetate solution and (6) drying the final catalyst product.

EP-A-417867 discloses a catalyst for the production of alcohols and/or carboxylic acid esters by hydrogenation of carboxylic acids or anhydrides, such catalyst comprising an alloy of at least one noble metal of Group VIII and at least one metal capable of alloying with the said Group VIII noble metal. Example 10 of that patent discloses such a hydrogenation catalyst comprising certain proportions of palladium, gold and rhenium.

The present invention provides a novel method for producing a catalyst which is useful for the vapour-phase manufacture of vinyl acetate from acetic acid, ethylene and oxygen which comprises three or four specific metals in specific amounts. The catalyst produced by this method is itself novel, as is evidenced by its properties.

Specifically, the present invention provides a method of preparing such a catalyst as defined in the claims hereto.

The invention also relates to the use of a catalyst prepared by such method for the production of vinyl acetate from acetic acid, ethylene and oxygen.

### DETAILED DESCRIPTION OF THE INVENTION

The catalyst of the present invention can be made by any of the prior art methods for the manufacture of catalysts useful for the manufacture of vinyl acetate by the reaction of ethylene, acetic acid, and oxygen. Of particular value are the methods described in U. S. Patents 3, 775,342; 5,314,858; or 5,332,710.

The support material for the catalyst according to the present invention can be of any diverse geometrical shape. For example, the support can be shaped as spheres, tablets, or cylinders having regular or irregular shapes. The geometrical dimensions of the support material can, in general, be in the range of 1-8 mm. A most suitable geometrical shape is, in particular, the spherical shape, for example, spheres with diameters in the range of 4-8 mm. These supports are generally called pills.

The specific surface area of the support material can vary within wide limits. For example, support materials which have an inner surface area of 50-300 m²/g and especially 100-200 m²/g (measured according to BET) are suitable.

Examples of support materials which can be used include silica, aluminum oxide, aluminum silicates or spinels. Silica is the preferred support material.

The active metal catalyst can be prepared by any of the above-described methods for the preparation of a palladium-gold catalyst wherein zirconium and or rhenium metal salts are added to the metal salt mixture used for impregnation of the catalyst support. The gold, palladium, and rhenium and/or zirconium salts in an aqueous solution are adsorbed into the pores of the support in what has been described in the prior art as the pore volume impregnation method; the treated supports are fixed to precipitate the metals into water-insoluble compounds; and the metal salts are reduced by treatment with a reducing agent, as for example ethylene or hydrazine.

In one method for the preparation of the catalyst of this invention, the appropriate quantities of water soluble salts of palladium, gold, and rhenium and/or zirconium are dissolved in a sufficient amount of water to provide a solution equivalent to about 90-110% of the pore volume of the porous support. Palladium (II) chloride, sodium palladium (II) chloride and palladium (II) nitrate are examples of suitable water-soluble palladium compounds, whereas auric (III) chloride or tetrachloroauric (III) acid and the alkali metal salts thereof can be used as the water-soluble gold compounds. The generally available tetrachloroauric (III) acid and the sodium palladium (II) chloride are preferred because of their high water solubility. Zirconium sulfate, zirconium nitrate, and zirconium chloride are typical of the salts of zirconium useful for the manufacture of the catalyst of this invention. Sodium perrhenate is an appropriate salt to use as a soluble salt for rhenium although rhenium (VII) heptoxide, or rhenium (III) chloride are also useful for the present invention. Typically, the quantity of these compounds employed is such as to provide from about 2 to about 14 grams/liter of palladium, from about 1 to about 8 grams/liter of gold, and from about 0.5 to about 4 grams/liter of zirconium, and about 1 to about 8 grams/liter of finished catalyst. Accordingly, the amount of gold present in the catalyst will be from about 10 to about 70% of the amount of palladium. The solution is adsorbed on the support, the support is dried, and the metals are fixed by immersing the support in an aqueous alkaline solution of sufficient concentration for a period of at least about 16 hours to cause the metal salts to become insoluble in water.

Alternatively, the fixing step may be carried out by immersing the treated supports in a sufficient volume of fixing solution to cover the bulk volume of the supports and causing the supports to be rotated as for example according to the method described in U. S. 5,332,710, which method is incorporated herewithin by reference. According to that reference, the impregnated supports are immersed in the alkaline solution and tumbled or rotated therein during the initial stages of precipitation of the water insoluble precious metal compounds such as to the oxides or hydroxides. The rotation or tumbling of the supports in the alkaline fixing solution should proceed for at least about 0.5 hour upon initial treatment and preferably for at least one hour. Rotation-immersion treatment can last as long as up to 4 hours. The treated supports may be allowed to stand in the fixing solution to ensure that complete precipitation of the water insoluble precious metal compounds takes place.

Any type of rotation, tumbling, or equivalent equipment which will keep the support in motion can be used as the exact apparatus utilized is not critical. What may be critical, however, is the extent of the motion. Thus, the motion should be sufficient so that all surfaces of the impregnated supports are evenly contacted with the alkaline fixing solution. The motion should not be so harsh that actual abrasion of the insoluble precious metal compounds takes place such that the insoluble compounds are abraded off the support surface. Generally, the extent of rotation should be about 1 to 10 rpm and possibly even higher depending upon the exact support utilized and the amount of precious metal to be deposited on the support. The rpm to be used is variable and may also depend upon the apparatus utilized, the size and shape of the support, the type of support, metal loadings, etc., but should fall within the guidelines expressed above. While a small amount of abrasion may take place, it is not to be such that the insoluble compounds are actually abraded off the support surface to an unacceptable degree.

The fixing solution is one which comprises an alkaline solution, for example, an aqueous solution which contains alkali metal hydroxides, alkali metal bicarbonates and/or alkali metal carbonates. It is particularly preferred to use aqueous solutions of sodium hydroxide or potassium hydroxide. Subsequent to the precipitation or fixing step, the supports are washed such as with distilled water so as to remove the anions, such as the chlorides, which are still contained on the support and freed from the initial impregnating solution by the precipitation process. Washing is continued until all of the anions are removed from the support. No more than about 1,000 ppm of anion should remain on the catalyst. To ensure substantially complete removal of the anions such as chloride ion from the catalyst, the wash effluent can be tested with silver nitrate. The catalyst is then dried at temperatures not to exceed about 150°C under an inert atmosphere such as a continuous nitrogen or air flow.

The fixed and washed material is then treated with a reducing agent in order to convert the metal salts and compounds which are present into the metallic form. The reduction can be carried out in the liquid phase, for example, with aqueous hydrazine hydrate, or in the gas phase, for example, with hydrogen or hydrocarbons, for example, ethylene. If the reduction is carried out with a solution of hydrazine hydrate, the reaction is preferably carried out at normal temperature. When the reduction is carried out in the gas phase, it can be advantageous to carry out the reaction at elevated temperature, for example, at 100-200°C in the case of reduction with ethylene. The reducing agent is appropriately employed in excess so that it is certain that all of the metal salts and compounds are converted into the metallic form.

In another method useful for the preparation of the improved catalyst of the present invention, a suitable catalyst support is first impregnated with an aqueous solution containing water-soluble palladium, gold, and either or both zirconium and rhenium compounds. Separate solutions of palladium, gold, and either or both rhenium and zirconium compounds could also be used successively, but it is less convenient to proceed in that fashion. The volume of solution used for impregnating the support with the metals is important. For effective deposition, the volume of the impregnating solution should be from 95 to 100% of the absorptive capacity of the catalyst support and preferably it should be 98-99%.

After impregnation of the support with the water soluble palladium, gold, and either or both zirconium and rhenium compounds, the impregnated supports are dried prior to fixing the palladium, gold compounds, and either or both zirconium and rhenium compounds, as water insoluble compounds on the support. The fixing step is divided into at least two separate stages of treatment with the alkaline fixing solution. In each separate fixing treatment, the amount of the alkaline reactive compound is no more than that equal to the molar amount required to react with all of the metal compound which is present on the support as a water soluble compound. Preferably, the amount of reactive compound used in each fixing stage is less than the molar amount required to react with all of the water soluble metal compound. The first fixing stage is conducted by impregnating the dried, impregnated support with the alkaline fixing solution in an amount equal to about the dry absorptivity of the support. The amount of the alkaline compound contained in solution should be such that the ratio of alkali metal to anion from the water soluble metal salt be from about 0.7:1 to about 1:1 molar and the volume should equal the dry support absorptivity. The alkaline fixing solution is simply poured onto the impregnated supports and the treated supports are allowed to stand for up to 24 hours or more during the precipitation. The second fixing stage is conducted by adding the undried partially fixed pills to a second portion of aqueous fixing solution. In this solution the ratio of alkali metal to anion from the metal salt should be from about 0.2:1 to about 0.9:1 molar. The volume of solution should cover the pills. Preferably, the total amount of alkali metal to anion should range from about 1.1:1 to about 1.6:1 molar for the combined fixing step. Subsequent to treatment in the first fixing stage, the treated supports are allowed to stand for a sufficient period of time to allow precipitation of the insoluble metal compounds. The period of time for this first fixing step will vary but typically will range from about 2 to about 8 hours before the support is again treated with the second portion of alkaline fixing solution.

Subsequent to treatment in the second fixing stage, the treated supports are allowed to stand again for at least an additional 2 hours, preferably, at least 4 hours and may stand to complete precipitation for up to about 16 hours.

The treatment in the second fixing stage can be equivalent to that of the first stage wherein the treated dried and partially fixed supports are impregnated with the fixing solution at the desired alkaline concentration. The total volume of solution is sufficient to cover the volume of the support.

Alternatively, the support can be impregnated in the second fixing stage by a process designated rotation immersion which is set forth in U. S. Patent 5,332,710. In this process, the once-fixed catalysts are immersed in the alkaline fixing solution and tumbled or rotated therein during the initial stages of the precipitation of the water insoluble metal compounds. The rotation or tumbling of the supports in the alkaline fixing solution should proceed for at least 0.5 hour upon the initial treatment and, preferably, for at least 1 hour. The rotation immersion treatment can last as long as up to 4 hours before the treated supports are allowed to stand in the fixing solution to insure that complete precipitation of the water soluble metal compounds take place.

As described above, any type of rotation or tumbling equipment can be used as the exact apparatus utilized is not critical. What may be critical, however, is the extent of the rotating motion. Thus, the rotation should be sufficient so that all surfaces of the impregnated supports are evenly contacted with the alkaline fixing solution. The rotation should not be so harsh that actual abrasion of the insoluble metal compounds takes place such that the insoluble compounds are abraded off the support surface. Generally, the extent of rotation should be about 1 to 10 rpm and possibly even higher depending upon the exact support utilized and the amount of metal to be deposited on the support. The rpm to be used is variable and may also depend upon the apparatus utilized, the size and shape of the support, the type of support, metal loadings, etc., but should fall within the guidelines expressed above that while a small amount of abrasion may take place, it is not to be such that the insoluble compounds are actually abraded off the support surface to an unacceptable degree.

The fixed pills are then reduced in a stream of ethylene gas or in a solution of hydrazine hydrate in a manner well known in the art.

Depending on the use for which the catalyst prepared in this way is intended, the latter can also be provided with customary additives. Thus, for example, additions of alkali metal acetates are advantageous when the catalyst is to be used for the preparation of unsaturated esters from olefins, oxygen and organic acids. In this case, for example, the catalyst can, for this purpose, be impregnated with an aqueous solution of potassium acetate and then dried.

The catalysts according to the invention can be used with particular advantage in the preparation of vinyl acetate from ethylene, oxygen and acetic acid in the gas phase. For this purpose, those catalysts according to the invention which contain silica as the support material and additives of alkali metal acetates are particularly suitable. In the above mentioned preparation of vinyl acetate, such catalysts are also distinguished by high activity and selectivity and by long life.

### EXAMPLE 1

Silica catalyst support, 250 g, provided by Sud Chemie having a spherical shape and a diameter of 7.3 mm is impregnated with 85 ml. of an aqueous solution containing 9.24g of an aqueous solution of sodium palladium chloride containing 18.95% palladium ions, 1.33g rhenium heptoxide, and 5.01g of an aqueous solution of sodium tetrachloroaurate containing 20.02% gold ions. The impregnated support is dried in hot air at a temperature not exceeding 100°C. The treated and dried support is impregnated with 85 ml an aqueous solution containing 1.76 g sodium hydroxide by soaking the pills in the solution. The volume of the sodium hydroxide solution is equal to the dry support absorptivity in the first fixing stage. After the first stage, the base-treated pills are allowed to stand for 4 hours and then subsequently poured into a second sodium hydroxide solution (250 ml of an aqueous solution containing 1.54 g NaOH). Subsequent to the second treatment, the base treated material is allowed to stand for an additional about 16 hours. After fixing, the base treated material is thoroughly washed with distilled water to remove the chloride ions to an acceptable level which is at most 1,000 ppm chloride. The water flow rate is about 200 cc/min for approximately 5 hours. The catalyst is dried under a continuous nitrogen flow at a temperature of no more than 150°C. The dried catalyst is reduced with a reducing gas containing 5% ethylene in nitrogen at a temperature of 150°C. The reducing gas is passed over the catalyst for 5 hours at atmospheric pressure. The reduced catalyst is impregnated with an aqueous solution containing 10 grams of potassium acetate at a solution volume equal to the dry absorptivity of the support. The catalyst is dried at a temperature of 150°C.

### EXAMPLE 2

The method of Example 1 is repeated using 1.59 g of zirconium sulfate instead of the rhenium heptoxide. In each of the fixing steps an aqueous solution of 2.24 g NaOH is used.

### EXAMPLE 3

The catalyst is prepared on the same support as used in Example 1. The support is impregnated with 85 ml of an aqueous solution containing 9.24 g of an aqueous solution of sodium palladium chloride containing 18.95% palladium ions, 1.59 g. zirconium sulfate, and 5.01 g of an aqueous solution of sodium tetrachloroaurate containing 20.02% gold ions. Precipitation is accomplished by adding 283 ml aqueous sodium hydroxide solution equivalent to 120% of the stoichiometric equivalent needed to convert the metal salts to their hydroxides. The flask is immediately rotated in a roto-evaporator (without vacuum) at approximately 5 rpm and rotation continued for 2.5 hours. After 2.5 hours, the rotation is stopped and the alkaline treated supports allowed to stand for an additional 16 hours to insure maximum precipitation of the metal salts as the insoluble hydroxides. The flask is drained and the alkaline treated material is washed with distilled water to remove the chloride ions. A water flow rate of about 200 cc per minute is used for approximately 5 hours. The catalyst is reduced with an aqueous solution of hydrazine hydrate (12/1 excess) at ambient room temperature for 4 hours. The pills are washed and dried for 1 hour at 100°C. The reduced catalyst is impregnated with an aqueous solution containing 10 grams of potassium acetate at a solution volume equal to the dry support absorbtivity and the catalyst is dried.

## Claims

1. A method of preparing a catalyst useful for the vapor-phase manufacture of vinyl acetate from acetic acid, ethylene and oxygen which consists of a porous support containing deposited thereupon metals comprising from about 2 to about 14 g/l of palladium, from about 1 to about 8 g/l of gold and at least one metal of the group consisting of about 0.5 to about 4 g/l of zirconium and from about 1 to about 8 g/l of rhenium, said method comprising: impregnating said support with water soluble compounds of said metals, converting said water soluble metal compounds to water insoluble metal compounds by immersing said impregnated support in a fixing solution containing a compound reactive with said water soluble compounds to precipitate on said support said water insoluble metal compounds and while said impregnated support is immersed in said fixing solution tumbling said impregnated support therein for a time sufficient to evenly coat the impregnated support with the fixing solution, completing precipitation of said water insoluble compounds, washing said support and reducing said water insoluble metal compounds to form free metals on said support.

2. The method of claim 1 wherein the catalyst consists of a porous support containing deposited thereupon metals comprising from about 2 to about 14 g/l of palladium, from about 1 to about 8 g/l of gold and from about 0.5 to about 4 g/l of zirconium.

3. The method of claim 1 wherein the catalyst consists of a porous support containing deposited thereupon metals comprising from about 2 to about 14 g/l of palladium, from about 1 to about 8 g/l of gold and from about 1 to about 8 g/l of rhenium.

4. The method of claim 1 wherein the catalyst consists of a porous support containing deposited thereupon metals comprising from about 2 to about 14 g/l of palladium, from about 1 to about 8 g/l of gold and from about 0.5 to about 4 g/l of zirconium and from about 1 to about 8 g/l of rhenium.

5. A method of preparing a catalyst useful for the vapor-phase manufacture of vinyl acetate from acetic acid, ethylene, and oxygen which consists of a porous support containing deposited thereupon metals comprising from about 2 to about 14 g/l of palladium, from about 1 to about 8 g/l of gold and at least one metal of the group consisting of about 0.5 to about 4 g/l of zirconium and from about 1 to about 8 g/l of rhenium comprising: contacting said support with at least one of said water soluble compounds of metal to impregnate the water soluble compound on the support, converting said water soluble metal compound(s) to water insoluble metal compound(s) by contacting said impregnated support in a first fixing step with a solution containing a compound reactive with said water soluble compound to precipitate on said support said water insoluble metal compound, contacting said first impregnated and fixed support with at least one other of said water soluble compounds of said metals to impregnate the water soluble compound(s) on the support, converting said second water soluble compound(s) of said metals to a water insoluble metal compound(s) by contacting said impregnated support in a second fixing step with a second solution containing a compound reactive with said second water soluble compound to precipitate on said support said second water insoluble metal compound, and thereafter reducing said water insoluble metal compounds to form free precious metals on said support.

6. The method of claim 5 wherein the catalyst consists of a porous support containing deposited thereupon metals comprising from about 2 to about 14 g/l of palladium, from about 1 to about 8 g/l of gold and from about 0.5 to about 4 g/l of zirconium.

7. The method of claim 5 wherein the catalyst consist of a porous support containing deposited thereupon metals comprising from about 2 to about 14 g/l of palladium, from about 1 to about 8 g/l of gold and from about 1 to about 8 g/l of rhenium.

8. The method of claim 5 wherein the catalyst consists of a porous support containing deposited thereupon metals comprising from about 2 to about 14 g/l of palladium, from about 1 to about 8 g/l of gold and from about 0.5 to about 4 g/l of zirconium and from about 1 to about 8 g/l of rhenium.

9. The use of the catalyst prepared by the method of any of the preceding claims for the production of vinyl acetate from acetic acid, ethylene and oxygen.

## Patentansprüche

1. Verfahren zur Herstellung eines Katalysators, der für die Herstellung von Vinylacetat in der Dampfphase aus Essigsäure, Ethylen und Sauerstoff geeignet ist und der aus einem porösen Träger besteht, auf dem Metalle abgeschieden sind, die etwa 2 bis etwa 14 g/l Palladium, etwa 1 bis etwa 8 g/l Gold und wenigstens ein Metall der Gruppe umfassen, die aus etwa 0,5 bis etwa 4 g/l Zirconium und etwa 1 bis etwa 8 g/l Rhenium besteht, wobei das Verfahren folgendes umfasst: Imprägnieren des Trägers mit wasserlöslichen Verbindungen der Metalle, Umwandeln der wasserlöslichen Metallverbindungen in wasserunlösliche Metallverbindungen durch Eintauchen des imprägnierten Trägers in eine Fixierlösung, die eine Verbindung enthält, die mit den wasserlöslichen Verbindungen so reagiert, dass auf dem Träger die wasserunlöslichen Metallverbindungen abgeschieden werden, und während der imprägnierte Träger in die Fixierlösung eingetaucht ist, Taumelnlassen des imprägnierten Trägers in derselben während einer ausreichenden Zeit, um den imprägnierten Träger gleichmäßig mit der Fixierlösung zu beschichten und die Ausfällung der wasserunlöslichen Verbindungen zu beenden, Waschen des Trägers und Reduzieren der wasserunlöslichen Metallverbindungen unter Bildung der freien Metalle auf dem Träger.

2. Verfahren gemäß Anspruch 1, wobei der Katalysator aus einem porösen Träger besteht, auf dem Metalle abgeschieden sind, die etwa 2 bis etwa 14 g/l Palladium, etwa 1 bis etwa 8 g/l Gold und etwa 0,5 bis etwa 4 g/l Zirconium umfassen.

3. Verfahren gemäß Anspruch 1, wobei der Katalysator aus einem porösen Träger besteht, auf dem Metalle abgeschieden sind, die etwa 2 bis etwa 14 g/l Palladium, etwa 1 bis etwa 8 g/l Gold und etwa 1 bis etwa 8 g/l Rhenium umfassen.

4. Verfahren gemäß Anspruch 1, wobei der Katalysator aus einem porösen Träger besteht, auf dem Metalle abgeschieden sind, die etwa 2 bis etwa 14 g/l Palladium, etwa 1 bis etwa 8 g/l Gold sowie etwa 0,5 bis etwa 4 g/l Zirconium und etwa 1 bis etwa 8 g/l Rhenium umfassen.

5. Verfahren zur Herstellung eines Katalysators, der für die Herstellung von Vinylacetat in der Dampfphase aus Essigsäure, Ethylen und Sauerstoff geeignet ist und der aus einem porösen Träger besteht, auf dem Metalle abgeschieden sind, die etwa 2 bis etwa 14 g/l Palladium, etwa 1 bis etwa 8 g/l Gold und wenigstens ein Metall der Gruppe umfassen, die aus etwa 0,5 bis etwa 4 g/l Zirconium und etwa 1 bis etwa 8 g/l Rhenium besteht, umfassend: In-Kontakt-Bringen des Trägers mit wenigstens einer der wasserlöslichen Metallverbindungen, so dass der Träger mit der wasserlöslichen Verbindung imprägniert wird, Umwandeln der wasserlöslichen Metallverbindungen in wasserunlösliche Metallverbindungen durch In-Kontakt-Bringen des imprägnierten Trägers in einem ersten Fixierschritt mit einer Lösung, die eine Verbindung enthält, die mit der wasserlöslichen Verbindung so reagiert, dass auf dem Träger die wasserunlösliche Metallverbindung abgeschieden wird, In-Kontakt-Bringen des ersten imprägnierten und fixierten Trägers mit wenigstens einer anderen der wasserlöslichen Metallverbindungen, so dass der Träger mit der wasserlöslichen Verbindung imprägniert wird, Umwandeln der zweiten wasserlöslichen Metallverbindungen in wasserunlösliche Metallverbindungen durch In-Kontakt-Bringen des imprägnierten Trägers in einem zweiten Fixierschritt mit einer zweiten Lösung, die eine Verbindung enthält, die mit der zweiten wasserlöslichen Verbindung so reagiert, dass auf dem Träger die zweite wasserunlösliche Metallverbindung abgeschieden wird, und danach Reduzieren der wasserunlöslichen Metallverbindungen unter Bildung freier Edelmetalle auf dem Träger.

6. Verfahren gemäß Anspruch 5, wobei der Katalysator aus einem porösen Träger besteht, auf dem Metalle abgeschieden sind, die etwa 2 bis etwa 14 g/l Palladium, etwa 1 bis etwa 8 g/l Gold und etwa 0,5 bis etwa 4 g/l Zirconium umfassen.

7. Verfahren gemäß Anspruch 5, wobei der Katalysator aus einem porösen Träger besteht, auf dem Metalle abgeschieden sind, die etwa 2 bis etwa 14 g/l Palladium, etwa 1 bis etwa 8 g/l Gold und etwa 1 bis etwa 8 g/l Rhenium umfassen.

8. Verfahren gemäß Anspruch 5, wobei der Katalysator aus einem porösen Träger besteht, auf dem Metalle abgeschieden sind, die etwa 2 bis etwa 14 g/l Palladium, etwa 1 bis etwa 8 g/l Gold sowie etwa 0,5 bis etwa 4 g/l Zirconium und etwa 1 bis etwa 8 g/l Rhenium umfassen.

9. Verwendung des nach dem Verfahren gemäß einem der vorstehenden Ansprüche hergestellten Katalysators zur Herstellung von Vinylacetat aus Essigsäure, Ethylen und Sauerstoff.

## Revendications

1. Procédé pour la préparation d'un catalyseur utile pour la production en phase vapeur d'acétate de vinyle à partir d'acide acétique, d'éthylène et d'oxygène, qui consiste en un support poreux contenant, à l'état déposé sur celui-ci, des métaux comprenant environ 2 à environ 14 g/l de palladium, environ 1 à environ 8 g/l d'or et au moins un métal du groupe consistant en une quantité d'environ 0,5 à environ 4 g/l de zirconium et une quantité d'environ 1 à environ 8 g/l de rhénium, ledit procédé comprenant les étapes consistant : à imprégner ledit support avec des composés hydrosolubles desdits métaux, à convertir lesdits composés métalliques hydrosolubles en composés métalliques insolubles dans l'eau en immergeant ledit support imprégné dans une solution fixatrice contenant un composé réactif avec lesdits composés hydrosolubles pour précipiter sur ledit support lesdits composés métalliques insolubles dans l'eau et, tandis que ledit support imprégné est immergé dans ladite solution fixatrice, à agiter au tambour ledit support imprégné qui se trouve dans cette solution pendant un temps suffisant pour revêtir uniformément le support imprégné avec la solution fixatrice, à achever la précipitation desdits composés insolubles dans l'eau, à laver ledit support et à réduire lesdits composés métalliques insolubles dans l'eau pour former les métaux libres sur ledit support.

2. Procédé suivant la revendication 1, dans lequel le catalyseur consiste en un support poreux contenant, à l'état déposé sur celui-ci, des métaux comprenant environ 2 à environ 14 g/l de palladium, environ 1 à environ 8 g/l d'or et environ 0,5 à environ 4 g/l de zirconium.

3. Procédé suivant la revendication 1, dans lequel le catalyseur consiste en un support poreux contenant, à l'état déposé sur celui-ci, des métaux comprenant environ 2 à environ 14 g/l de palladium, environ 1 à environ 8 g/l d'or et environ 1 à environ 8 g/l de rhénium.

4. Procédé suivant la revendication 1, dans lequel le catalyseur consiste en un support poreux contenant, à l'état déposé sur celui-ci, des métaux comprenant environ 2 à environ 14 g/l de palladium, environ 1 à environ 8 g/l d'or et environ 0,5 à environ 4 g/l de zirconium et environ 1 à environ 8 g/l de rhénium.

5. Procédé pour la préparation d'un catalyseur utile pour la production en phase vapeur d'acétate de vinyle à partir d'acide acétique, d'éthylène et d'oxygène, qui consiste en un support poreux contenant, à l'état déposé sur celui-ci, des métaux comprenant environ 2 à environ 14 g/l de palladium, environ 1 à environ 8 g/l d'or et au moins un métal du groupe consistant en une quantité d'environ 0,5 à environ 4 g/l de zirconium et une quantité d'environ 1 à environ 8 g/l de rhénium, comprenant les étapes consistant : à mettre en contact ledit support avec au moins un des composés métalliques hydrosolubles pour imprégner le support avec le composé hydrosoluble, à convertir ledit ou lesdits composés métalliques hydrosolubles en un ou plusieurs composés métalliques insolubles dans l'eau en mettant en contact ledit support imprégné dans une première étape de fixage avec une solution contenant un composé réactif avec ledit composé hydrosoluble pour précipiter sur ledit support ledit composé métallique insoluble dans l'eau, à mettre en contact ledit premier support imprégné et fixé avec au moins un autre desdits composés hydrosolubles desdits métaux pour imprégner le support avec ledit ou lesdits composés hydrosolubles, à convertir ledit ou lesdits seconds composés hydrosolubles desdits métaux en un ou plusieurs composés métalliques insolubles dans l'eau en mettant en contact ledit support imprégné dans une seconde étape de fixage avec une seconde solution contenant un composé réactif avec ledit second composé hydrosoluble pour précipiter sur ledit support ledit second composé métallique insoluble dans l'eau, puis à réduire lesdits composés métalliques insolubles dans l'eau pour former des métaux précieux libres sur ledit support.

6. Procédé suivant la revendication 5, dans lequel le catalyseur consiste en un support poreux contenant, à l'état déposé sur celui-ci, des métaux comprenant environ 2 à environ 14 g/l de palladium, environ 1 à environ 8 g/l d'or et environ 0,5 à environ 4 g/l de zirconium.

7. Procédé suivant la revendication 5, dans lequel le catalyseur consiste en un support poreux contenant, à l'état déposé sur celui-ci, des métaux comprenant environ 2 à environ 14 g/l de palladium, environ 1 à environ 8 g/l d'or et environ 1 à environ 8 g/l de rhénium.

8. Procédé suivant la revendication 5, dans lequel le catalyseur consiste en un support poreux contenant, à l'état déposé sur celui-ci, des métaux comprenant environ 2 à environ 14 g/l de palladium, environ 1 à environ 8 g/l d'or et environ 0,5 à environ 4 g/l de zirconium et environ 1 à environ 8 g/l de rhénium.

9. Utilisation du catalyseur préparé par le procédé suivant l'une quelconque des revendications précédentes pour la production d'acétate de vinyle à partir d'acide acétique, d'éthylène et d'oxygène.
